# EUROPEAN PATENT SPECIFICATION

(11) **EP 3 981 468 B1**
(45) Date of publication and mention of the grant of the patent: **30.09.2026**
(21) Application number: 20826284.0
(22) Date of filing: 19.06.2020
(51) Int. Cl.: A61K 31/20, A61P 9/14

(54) **TRICAPRIN FOR USE IN PREVENTING AORTIC ANEURYSM**
TRICAPRIN ZUR VERWENDUNG BEI DER VORBEUGUNG VON AORTENANEURYSMAEN
TRICAPRIN POUR L'UTILISATION À LA PRÉVENTION DE L'ANÉVRISME AORTIQUE

(30) Priority: 21.06.2019 JP 2019115804
(43) Date of publication of application: 13.04.2022
(73) Proprietor: Kinki University, Higashi-Osaka-shi Osaka 577-8502 (JP); Yashiro Co., Ltd., Osaka-shi, Osaka 547-0003 (JP); Osaka University, Suita-shi, Osaka 565-0871 (JP)
(72) Inventor: ZAIMA Nobuhiro, Nara City, Nara 6318505 (JP); KUGO Hirona, Nara City, Nara 6318505 (JP); KITANO Motohiro, Osaka-shi, Osaka 547-0003 (JP); HIROTA Yoshinori, Osaka-shi, Osaka 547-0003 (JP); HIRANO Ken-ichi, Suita-shi, Osaka 565-0871 (JP)
(74) Representative: Gill Jennings & Every LLP
(86) International application number: PCT/JP2020/024099
(87) International publication number: WO 2020/256095

(56) References cited:
- WO-A1-2011/007565
- WO-A1-2013/031729
- WO-A1-2015/005393
- WO-A1-2015/050071
- WO-A1-2021/235044

## Description

### Field

The present invention relates to a processed food (health food) and a pharmaceutical composition for use in preventing an aortic aneurysm. The invention is as defined in the claims. Background

Among blood vessels originating at the heart, the largest blood vessel initially forms the ascending aorta heading upward, leads into the arch artery which bends in an arch shape and turns around the neck, and then leads into the descending aorta heading downward. The abdominal aorta is a downstream segment of the descending aorta passing through the thoracic diaphragm.

The abdominal aortic aneurysm (hereinafter also abbreviated to "AAA") is a condition where there is localized enlargement of the abdominal aorta. Specifically, AAA refers to a state in which the aorta, which usually has a diameter of about 20 mm, dilates to a diameter of 30 mm or more.

The rupture of an abdominal aortic aneurysm results in a drop in blood pressure and a sudden shock state. The dilation of the abdominal aorta is associated with no noticeable symptoms in many cases, and, if it is ruptured, the fatality rate is reported to be more than 80%.

Currently, the abdominal aortic aneurysm is accidentally found by physical examination or the like, and undergoing a preventive surgery before the rupture is considered the only available treatment method. Thus, it can be said that society demands a therapeutic agent or prophylactic agent for an abdominal aortic aneurysm.

Patent Literature 1 describes an agent for preventing and/or treating the progression of an aortic aneurysm including a compound having PPARg inhibitory activity as an active component. Upon a circulatory failure inside the blood vessel wall, fibroblasts constituting the tunica adventitia of the abdominal aortic aneurysm express PPARγ and differentiate into adipocyte-like cells, thereby causing abnormal accumulation of triglycerides in the blood vessel wall and weakening the blood vessel wall. Thus, it is expected that inhibiting PPARγ prevents the occurrence, progression, or rupture of an aortic aneurysm and improves the vital prognosis of patients with aortic aneurysms.

Furthermore, Patent Literature 2 discloses a prophylactic or therapeutic agent for an aortic aneurysm including eicosapentaenoic acid or docosahexaenoic acid as an active component.

WO 2015/050071 A1 discloses a nutritional supplement that is a combination of a highly unsaturated fatty acid and a medium chain fatty acid in the context of improving cardiovascular disease.

WO 2013/031729 A1 relates to medium chain fatty acids having utility in the treatment of atherosclerosis.

### Citation List

### Patent Literature

Patent Literature 1: International Publication No. 2011/007565
Patent Literature 2: International Publication No. 2015/005393
Patent Literature 3: Japanese Patent Application Laid-Open No. 2012-235715

### Summary

### Technical Problem

It is shown that eicosapentaenoic acid and docosahexaenoic acid described in Patent Literature 2 have an effect of inhibiting progression of an aortic aneurysm. However, such an effect is limited and there has been a demand for a composition used in an agent or processed food for further inhibiting the progression of an aortic aneurysm. Solution to Problem

The present invention has been made in view of the above-mentioned problems. More specifically, a composition for use in preventing an aortic aneurysm according to the present invention is characterized by including a medium chain fatty acid as an active component, wherein the medium chain fatty acid is in the form of tricaprin. Furthermore, the composition for use in preventing an aortic aneurysm according to the present invention can be prepared as a pharmaceutical composition for use in preventing an aortic aneurysm and a processed food composition for use in preventing an aortic aneurysm.

### Advantageous Effects of Invention

The composition for use in preventing an aortic aneurysm according to the present invention can effectively inhibit the progression of an aortic aneurysm. Thus, when an aortic aneurysm is found by examination or the like, for example, administering the composition can inhibit the progression of an aortic aneurysm. This treatment provides an effect of eliminating the need of, for example, performing a surgery for inserting a stent graft. Furthermore, the composition for use in preventing an aortic aneurysm according to the present invention can prevent the rupture of an aortic aneurysm and thus has an effect of improving the vital prognosis.

In a first aspect, the invention provides a pharmaceutical composition for use in preventing an aortic aneurysm, comprising a medium chain fatty acid as an active component, wherein the medium chain fatty acid is in the form of tricaprin.

In a second aspect, the invention provides a processed food for use in preventing an aortic aneurysm, comprising a medium chain fatty acid as an active component, wherein the medium chain fatty acid is in the form of tricaprin.

### Brief Description of Drawings

Fig. 1 is a graph showing survival rate in a control group, a tricaprylin administration group, and a tricaprin administration group during a test period.
Fig. 2 is a graph showing results in which the diameter of the aorta is measured in the control group, the tricaprylin administration group, and the tricaprin administration group after completion of the test.

### Description of Embodiments

Hereinafter, a composition for use in preventing an aortic aneurysm (a pharmaceutical composition for use in preventing an aortic aneurysm and a processed food composition for use in preventing an aortic aneurysm) according to the present invention will be described with reference to Examples.

In the present invention, preventing an aortic aneurysm means preventing the occurrence of an aortic aneurysm and inhibiting its progression whether in the abdomen or the chest by the action of inhibiting the dilation of the aorta and inhibiting the progression of the aortic aneurysm. Note that the progression includes rupture of an aortic aneurysm.

The composition for use in preventing an aortic aneurysm according to the present invention comprises a medium chain fatty acid as a main component, wherein the medium chain fatty acid is in the form of tricaprin. Medium chain fatty acids as described herein are saturated fatty acids having 5 to 12 carbon atoms. Specifically, the medium chain fatty acid is in the form of tricaprin. Saturated fatty acids include pentanoic acid (C5: valeric acid), hexanoic acid (C6: caproic acid), heptanoic acid (C7: enanthic acid), octanoic acid (C8: caprylic acid), nonanoic acid (C9: pelargonic acid), decanoic acid (C10: capric acid), and dodecanoic acid (C12: lauric acid).

These medium chain fatty acids may be used solely or as a mixture of two or more kinds thereof. Furthermore, the medium chain fatty acid used in the composition for use in preventing an aortic aneurysm according to the present invention may be naturally derived or artificially synthesized. For example, it is reported that coconut and palm fruit contain medium chain fatty acids at a content of nearly 60%.

Note that the medium chain fatty acid may be oils and fats (in the form of triglycerides or diglycerides). This is because triglycerides and diglycerides, which are esters derived from three fatty acids bonded to glycerol and two fatty acids bonded to glycerol, respectively, are broken down into glycerol and fatty acids by enzyme lipases and absorbed in the intestine. Further, the oils and fats may be esters derived from medium chain fatty acids having different number of carbon atoms and glycerol. However, esters derived from medium chain fatty acids having the same number of carbon atoms and glycerol are more preferable.

In a case where the composition for use in preventing an aortic aneurysm according to the present invention is used as the pharmaceutical composition for use in preventing an aortic aneurysm, components other than the medium chain fatty acid may be included. For example, a carrier, an excipient, a binder, a disintegrant, a lubricant, a colorant, and other components, which are suitable for the use applications of the present invention, can be suitably used.

Examples of the carrier and the excipient include lactose, glucose, sucrose, mannitol, potato starch, corn starch, calcium carbonate, calcium phosphate, calcium sulfate, and crystalline cellulose.

As the binder, starch, gelatin, syrup, tragacanth rubber, polyvinyl alcohol, polyvinyl ether, polyvinylpyrrolidone, hydroxypropyl cellulose, methyl cellulose, ethyl cellulose, and carboxymethyl cellulose may be used.

As the disintegrant, starch, agar, gelatin powder, crystalline cellulose, calcium carbonate, sodium hydrogen carbonate, sodium alginate, sodium carboxymethyl cellulose, and calcium carboxymethyl cellulose may be used.

Examples of the lubricant include magnesium stearate, hydrogenated vegetable oils, talc, and macrogol.

As the colorant, a colorant that is allowed to be added to pharmaceutical compositions may be used.

The pharmaceutical composition for use in preventing an aortic aneurysm of the present invention may be coated with one or more layers of sucrose, gelatin, purified shellac, gelatin, glycerin, sorbitol, ethyl cellulose, hydroxypropyl cellulose, hydroxypropyl methyl cellulose, polyvinylpyrrolidone, cellulose acetate phthalate, hydroxypropyl methyl cellulose phthalate, methyl methacrylate, and a methacrylic acid polymer. As necessary, a pH regulator, a buffer, a stabilizer, a solubilizer and the like may be added.

Furthermore, the pharmaceutical composition for use in preventing an aortic aneurysm of the present invention can be provided as a formulation in any form. The present invention can be provided as a formulation for oral administration including in the form of, for example, a tablet such as a sugar-coated tablet, a buccal tablet, a coating tablet, or a chewable tablet, a troche, a pill, a powder, a capsule such as a soft capsule, a granule, a suspension, an emulsion, syrup such as dry syrup, and a liquid such as an elixir.

Furthermore, the present invention can be provided as a formulation for parenteral administration. Examples of the parenteral administration include intravenous injection, subcutaneous injection, intraperitoneal injection, intramuscular injection, transdermal administration, nasal administration, transpulmonary administration, enteral administration, buccal administration, and transmucosal administration. For example, an injection, a transdermal absorption tape, an aerosol preparation, a suppository, and the like can be mentioned.

Further, the pharmaceutical composition for use in preventing an aortic aneurysm of the present invention can be produced by using any method. For example, the pharmaceutical composition for use in preventing an aortic aneurysm of the present invention can be produced by using a known production method to mix a medium chain fatty acid with various materials described above to obtain desired contents and then form the resulting mixture into a formulation in a desired form.

Furthermore, the composition for use in preventing an aortic aneurysm of the present invention may be provided in the form of foods and drinks as the processed food composition for use in preventing an aortic aneurysm. The processed food composition for use in preventing an aortic aneurysm comprises a medium chain fatty acid as an active component, wherein the medium chain fatty acid is in the form of tricaprin. The processed food for use in preventing an aortic aneurysm may be composed of only the medium chain fatty acid or the oils and fats serving as an active component. For example, general foods, foods for specified health use, food with nutrient function claims, dietary supplements, functional foods, medical foods, and foods for the elderly can be mentioned. Furthermore, for example, the present invention can be provided as foods and drinks labelled as the foods for specified health use and those labelled with an effect of preventing or improving an aortic aneurysm.

Furthermore, the composition for use in preventing an aortic aneurysm of the present invention can be used as a food material for use in preventing an aortic aneurysm, which is added to, mixed with, or coated on other foods. In particular, as food for specified health use, a tablet including at least a binder and a medium chain fatty acid or oils and fats with a medium chain fatty acid content of 20% by mass or more is preferable. Note that the content may be reduced to one third of this amount in a case where the composition is provided as the oils and fats.

Furthermore, it is reported that the aortic aneurysm is caused by excessive intake of high-fat meats. Thus, it is preferable that the composition for use in preventing an aortic aneurysm be included in a sauce or paste (dipping sauce) for meat in a predetermined amount or more, and this sauce or paste can then be used as the processed food composition for use in preventing an aortic aneurysm according to the present invention. These processed foods can be prepared by adding a predetermined amount of the prophylactic composition to a known sauce material or dipping sauce material.

For example, in a case of the sauce for grilled meat, the processed food composition for use in preventing an aortic aneurysm of the present invention can be prepared by adding a predetermined amount (desirably 20% by mass) or more of the composition for use in preventing an aortic aneurysm according to the present invention to dipping sauce materials including onion, ginger, garlic, soy source, sake, sesame oil, miso, sugar, sesame, and the like.

### Examples

### (Example 1)

### <Animal model preparation method>

SD-male rats of 4 weeks old (70 g to 90 g) were used. Eighteen rats and eight rats were prepared as a control group and a medium chain fatty acid administration group, respectively.

Upon arrival, all rats were preliminarily fed normal feed without additional supplements for one week. Then, the medium chain fatty acid administration group was fed normal feed and forcibly administered with high-content neutral fats (decanoic acid occupied 98% of constituent fatty acids) in an amount of 1,145 mg/kg body-weight/day. The control group was fed normal feed and forcibly administered with water in an amount of 1,145 mg/kg body-weight/day.

In this test, the medium chain fatty acid was fed in the form of tricaprin. Tricaprin is a substance in which decanoic acid (C10), a linear saturated fatty acid having 10 carbon atoms, is bonded to glycerol.

From the second week after the arrival, all rats were fed high-fat feed. Then, the medium chain fatty acid administration group was forcibly administered with the high-content neutral fats in an amount of 1,145 mg/kg bodyweight/day. Furthermore, the control group was forcibly administered with water in an amount of 1,145 mg/kg bodyweight/day.

Three weeks after the arrival, an abdominal aortic aneurysm induction treatment was performed. In the abdominal aortic aneurysm induction treatment, a catheter is inserted in the rat abdominal aorta and the inserted catheter is ligated with the abdominal aorta. The treatment was performed according to the method disclosed in Patent Literature 3.

After that, for the next four weeks, the control group had been fed the high-fat feed and forcibly administered with water in an amount of 1,145 mg/kg body-weight/day. Furthermore, the medium chain fatty acid administration group had been fed the high-fat feed and forcibly administered with the high-content neutral fats. Then, four weeks after the abdominal aortic aneurysm induction treatment, the rats were euthanized and dissected.

### <Test results>

Table 1 shows results from examining whether or not abdominal aortic aneurysms occurred in the control group and the medium chain fatty acid administration group. Note that the abdominal aortic aneurysm was determined to have occurred if the blood vessel at the site where the abdominal aortic aneurysm induction treatment was performed dilated to twice or more its original thickness.

Referring to Table 1, abdominal aortic aneurysms occurred in 7 out of 18 rats in the control group. The occurrence of the abdominal aortic aneurysm could not be confirmed in 11 rats in the control group.

On the other hand, the occurrence of the abdominal aortic aneurysm was observed in 1 rat in the medium chain fatty acid administration group. The occurrence of the abdominal aortic aneurysm could not be confirmed in 7 rats in the medium chain fatty acid administration group. Based on the above results, the frequency of occurrence of abdominal aortic aneurysms was 38.9% in the control group and 12.5% in the medium chain fatty acid administration group.

**[Table 1]**

| **Frequency of occurrence of abdominal aortic aneurysm** | | | |
|---|---|---|---|
| | **Occurrence not confirmed** | **Occurrence confirmed** | **Frequency of occurrence (%)** |
| **Contron group** | 11 | 7 | 38.9 |
| **Medium chain fatty acid administration group** | 7 | 1 | 12.5 |

Table 2 shows a result examining whether or not the abdominal aortic aneurysm was ruptured in rats with observed abdominal aortic aneurysms. Referring to Table 2, the rupture of the abdominal aortic aneurysm was confirmed in 2 out of 7 rats in the control group. The rupture of the abdominal aortic aneurysm was not observed in the remaining 5 rats in the control group.

On the other hand, the rupture of the abdominal aortic aneurysm was not observed in a rat with observed aortic aneurysm in the medium chain fatty acid administration group.

Based on the above results, a rupture rate of the abdominal aortic aneurysm was 28.6% in the control group and 0.0% in the medium chain fatty acid administration group.

**[Table 2]**

| **Rupture rate of abdominal aortic aneurysm** | | | |
|---|---|---|---|
| | **Rapture not confirmed** | **Rapture confirmed** | **Rupture rate (%)** |
| **Contron group** | 5 | 2 | 28.6 |
| **Medium chain fatty acid administration group** | 1 | 0 | 0 |

### (Example 2)

Next, test results obtained by using tricaprin (an ester of three decanoic acids and glycerol) and tricaprylin (an ester of three octanoic acids of C8 and glycerol) as the medium chain fatty acid is shown. The animal model preparation method is the same as that in Example 1. A group in which tricaprin (C10) was given is referred to as a tricaprin administration group and a group in which tricaprylin (C8) was given is referred to as a tricaprylin administration group.

Twenty-three, eleven, and eighteen model animals were prepared as a control group, the tricaprylin administration group, and the tricaprin administration group, respectively.

Table 3 shows results from examining whether or not the abdominal aortic aneurysm occurred in the control group, the tricaprylin administration group, and the tricaprin administration group. Note that the abdominal aortic aneurysm was determined to have occurred if the blood vessel at the site where the abdominal aortic aneurysm induction treatment was performed dilated to twice or more its original thickness.

Referring to Table 3, the abdominal aortic aneurysms occurred in 7 out of 23 rats in the control group. The occurrence of the abdominal aortic aneurysm could not be confirmed in 16 rats in the control group.

On the other hand, the occurrence of the abdominal aortic aneurysm was confirmed in 3 out of 11 rats in the tricaprylin administration group. Furthermore, the occurrence of the abdominal aortic aneurysm could not be confirmed in the remaining 8 rats. The occurrence of the abdominal aortic aneurysm was confirmed in 1 out of 18 rats in the tricaprin administration group. Furthermore, the occurrence of the abdominal aortic aneurysm could not be confirmed in the remaining 17 rats.

To make comparison, the frequency of occurrence of the control group was determined to be 30.4% and the tricaprylin administration, 27.3%. Thus, the tricaprylin administration group had the lower frequency of occurrence compared to the control group. The frequency of occurrence in the tricaprin administration group was 5.6%, which was drastically lower than that in the control group.

**[Table 3]**

| **Frequency of occurrence of abdominal aortic aneurysm** | | | |
|---|---|---|---|
| | **Occurrence not confirmed** | **Occurrence confirmed** | **Frequency of occurrence (%)** |
| **Contron group** | 16 | 7 | 30.4 |
| **Tricaprylin administration group** | 8 | 3 | 27.3 |
| **Tricaprin administration group** | 17 | 1 | 5.6 |

Table 4 shows results from examining whether or not the aortic aneurysms ruptured in rats with observed abdominal aortic aneurysms in Table 3. Referring to Table 4, the rupture of the abdominal aortic aneurysm was confirmed in 2 out of 7 rats with observed abdominal aortic aneurysms in the control group. The rupture of the abdominal aortic aneurysm was not observed in the remaining 5 rats.

On the other hand, in the tricaprylin administration group, the rupture of the abdominal aortic aneurysm was confirmed in 1 out of 3 rats with observed abdominal aortic aneurysms and the rupture of the abdominal aortic aneurysm was not observed in the remaining 2 rats. In the tricaprin administration group, the rupture of the abdominal aortic aneurysm was not confirmed in the 1 rat in which the abdominal aortic aneurysm occurred.

When measurements were converted into rupture rates (%), the control group measured 28.6%, the tricaprylin administration group measured 33.3%, and the tricaprin administration group measured 0.0%.

**[Table 4]**

| **Rupture rate of abdominal aortic aneurysm** | | | |
|---|---|---|---|
| | **Occurrence not confirmed** | **Occurrence confirmed** | **Frequency of occurrence (%)** |
| **Contron group** | 5 | 2 | 28.6 |
| **Tricaprylin administration group** | 2 | 1 | 33.3 |
| **Tricaprin administration group** | 1 | 0 | 0 |

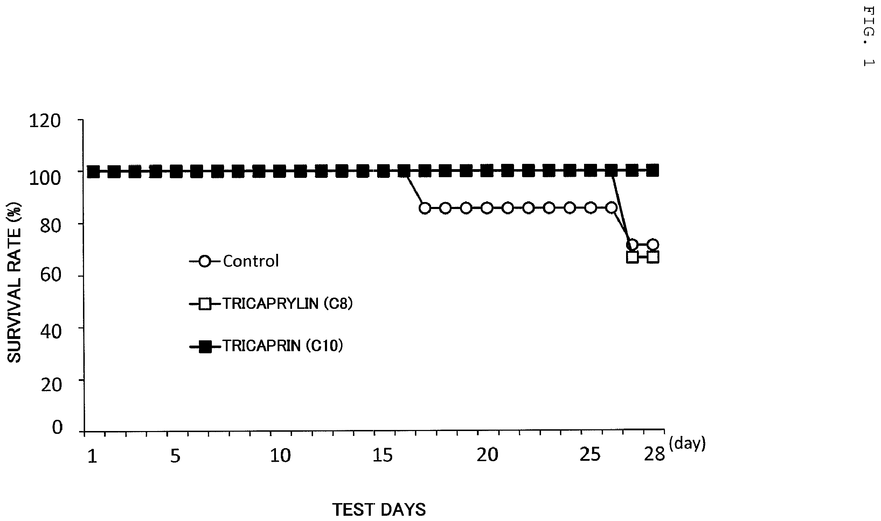

Fig. 1 shows a survival rate (%) in this test. The horizontal axis indicates the number of days (day) from Day 0 when the abdominal aortic aneurysm induction treatment was performed, and the vertical axis indicates the survival rate (%). The survival rate declined on Day 17 in the control, while the survival rate remained 100% until Day 26 in the tricaprylin administration group. According to this result, the tricaprylin administration group, despite having the higher rupture ratio compared to the control group in Table 4, had the higher survival rate, indicating the effect of administering tricaprylin. The tricaprin administration group maintained the 100% survival rate during the test period.

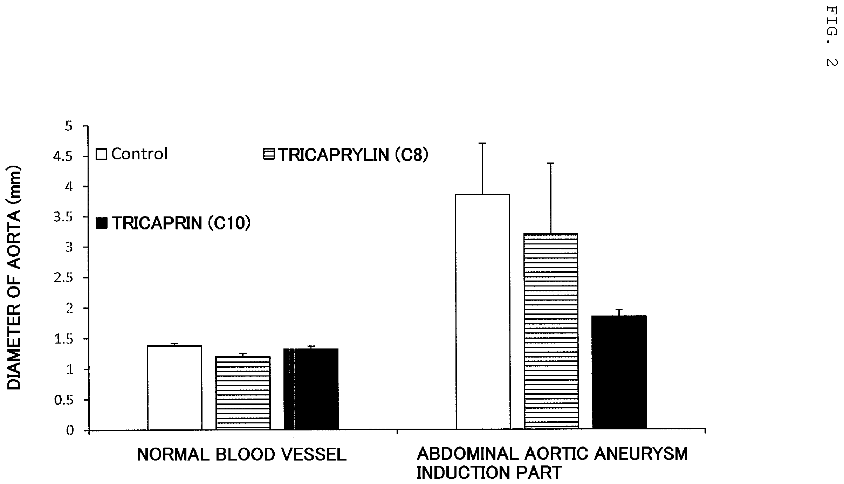

Fig. 2 shows a measurement result of a diameter (mm) of the aorta. Referring to Fig. 2, the horizontal axis indicates which measurements are from the normal blood vessel and which are from the blood vessel at the abdominal aortic aneurysm induction part; the vertical axis indicates the diameter (mm) of the aorta. The normal blood vessel refers to the normal aorta at the upper and lower sides of the part in which the abdominal aortic aneurysm was induced.

The thickness of the normal blood vessel was practically the same (from 1.5 mm to 1.8 mm) between the control group, the tricaprylin administration group, and the tricaprin administration group. However, the diameter of the aorta in the abdominal aortic aneurysm induction part was about 4 mm in the control group, about 3.5 mm in the tricaprylin administration group, and about 2.2 mm in the tricaprin administration group, showing that both administration groups had a lower degree of dilation compared to the control group.

Based on the above results, it was found that intake of the medium chain fatty acid could significantly reduce the frequency of occurrence and the rupture of the abdominal aortic aneurysm.

### Industrial Applicability

The pharmaceutical composition and processed food for use in preventing an aortic aneurysm according to the present invention can be suitably used for use in preventing an aortic aneurysm and inhibiting progression of the aortic aneurysm.

## Claims

1. A pharmaceutical composition for use in preventing an aortic aneurysm, comprising a medium chain fatty acid as an active component, wherein the medium chain fatty acid is in the form of tricaprin.

2. The pharmaceutical composition for use according to claim 1, further comprising a pharmaceutically acceptable carrier, excipient, binder, disintegrant, lubricant, or colorant.

3. The pharmaceutical composition for use according to claim 1 or 2, wherein the pharmaceutical composition is formulated for oral administration.

4. The pharmaceutical composition for use according to claim 3, wherein the oral administration is in the form of a tablet, a troche, a capsule, a powder, a granule, a suspension, a liquid, an emulsion, or a syrup.

5. The pharmaceutical composition for use according to claim 4, wherein the liquid is an elixir.

6. The pharmaceutical composition for use according to claim 1 or 2, wherein the pharmaceutical composition is formulated for parenteral administration.

7. The pharmaceutical composition for use according to claim **6,** wherein the parenteral administration is intravenous injection, subcutaneous injection, intraperitoneal injection, intramuscular injection, transdermal administration, nasal administration, transpulmonary administration, enteral administration, buccal administration, or transmucosal administration.

8. A processed food for use in preventing an aortic aneurysm, comprising a medium chain fatty acid as an active component, wherein the medium chain fatty acid is in the form of tricaprin.

9. The processed food for use according to claim 8, wherein the processed food is a sauce or paste.

10. The processed food or pharmaceutical composition for use according to any preceding claim, comprising 20% by mass or more of tricaprin.

## Patentansprüche

1. Pharmazeutische Zusammensetzung zur Verwendung bei der Vorbeugung eines Aortenaneurysmas, umfassend eine mittelkettige Fettsäure als Wirkstoff, wobei die mittelkettige Fettsäure in Form von Tricaprin vorliegt.

2. Pharmazeutische Zusammensetzung zur Verwendung nach Anspruch 1, weiter umfassend einen pharmazeutisch annehmbaren Träger, Hilfsstoff, Bindemittel, Sprengmittel, Gleitmittel oder Farbstoff.

3. Pharmazeutische Zusammensetzung zur Verwendung nach Anspruch 1 oder 2, wobei die pharmazeutische Zusammensetzung zur oralen Verabreichung formuliert ist.

4. Pharmazeutische Zusammensetzung zur Verwendung nach Anspruch 3, wobei die orale Verabreichung in Form einer Tablette, einer Pastille, einer Kapsel, eines Pulvers, eines Granulats, einer Suspension, einer Flüssigkeit, einer Emulsion oder eines Sirups erfolgt.

5. Pharmazeutische Zusammensetzung zur Verwendung nach Anspruch 4, wobei die Flüssigkeit ein Elixier ist.

6. Pharmazeutische Zusammensetzung zur Verwendung nach Anspruch 1 oder 2, wobei die pharmazeutische Zusammensetzung zur parenteralen Verabreichung formuliert ist.

7. Pharmazeutische Zusammensetzung zur Verwendung nach Anspruch 6, wobei die parenterale Verabreichung eine intravenöse Injektion, eine subkutane Injektion, eine intraperitoneale Injektion, eine intramuskuläre Injektion, eine transdermale Verabreichung, eine nasale Verabreichung, eine transpulmonale Verabreichung, eine enterale Verabreichung, eine bukkale Verabreichung oder eine transmukosale Verabreichung ist.

8. Verarbeitetes Lebensmittel zur Verwendung bei der Vorbeugung eines Aortenaneurysmas, umfassend eine mittelkettige Fettsäure als Wirkstoff, wobei die mittelkettige Fettsäure in Form von Tricaprin vorliegt.

9. Verarbeitetes Lebensmittel zur Verwendung nach Anspruch 8, wobei es sich bei dem verarbeiteten Lebensmittel um eine Soße oder Paste handelt.

10. Verarbeitetes Lebensmittel oder pharmazeutische Zusammensetzung zur Verwendung nach einem der vorstehenden Ansprüche, umfassend 20 Massen-% oder mehr an Tricaprin.

## Revendications

1. Composition pharmaceutique destinée à être utilisée pour prévenir un anévrisme aortique, comprenant un acide gras à chaîne moyenne en tant que composant actif, dans laquelle l'acide gras à chaîne moyenne est sous forme de tricaprine.

2. Composition pharmaceutique destinée à être utilisée selon la revendication 1, comprenant en outre un support, excipient, liant, désintégrant, lubrifiant ou colorant pharmaceutiquement acceptable.

3. Composition pharmaceutique destinée à être utilisée selon la revendication 1 ou la revendication 2, dans laquelle la composition pharmaceutique est formulée pour une administration orale.

4. Composition pharmaceutique destinée à être utilisée selon la revendication 3, dans laquelle l'administration orale est sous forme de comprimé, de pastille, de capsule, de poudre, de granulé, de suspension, de liquide, d'émulsion, ou de sirop.

5. Composition pharmaceutique destinée à être utilisée selon la revendication 4, dans laquelle le liquide est un élixir.

6. Composition pharmaceutique destinée à être utilisée selon la revendication 1 ou la revendication 2, dans laquelle la composition pharmaceutique est formulée pour une administration parentérale.

7. Composition pharmaceutique destinée à être utilisée selon la revendication 6, dans laquelle l'administration parentérale est l'injection intraveineuse, l'injection sous-cutanée, l'injection intrapéritonéale, l'injection intramusculaire, l'administration transdermique, l'administration nasale, l'administration transpulmonaire, l'administration entérale, l'administration buccale ou l'administration par voie transmuqueuse.

8. Aliment transformé destiné à être utilisé pour prévenir un anévrisme aortique, comprenant un acide gras à chaîne moyenne en tant que composant actif, dans lequel l'acide gras à chaîne moyenne est sous forme de tricaprine.

9. Aliment transformé destiné à être utilisé selon la revendication 8, dans lequel l'aliment transformé est une sauce ou une pâte.

10. Aliment transformé ou composition pharmaceutique destiné(e) à être utilisé(e) selon une quelconque revendication précédente, comprenant 20 % en masse ou plus de tricaprine.
